# EUROPÄISCHE PATENTANMELDUNG

(11) **EP 3 736 591 A1**
(43) Veröffentlichungstag der Anmeldung: **11.11.2020**
(21) Anmeldenummer: 19173594.3
(22) Anmeldetag: 09.05.2019
(51) Int. Cl.: G01R 33/38, A61B 5/055

(54) **GRUNDFELDMAGNETANORDNUNG FÜR EIN MAGNETRESONANZTOMOGRAPHIE-SYSTEM**

(71) Anmelder: Siemens Healthcare GmbH, 91052 Erlangen (DE)
(72) Erfinder: Popescu, Stefan, 91056 Erlangen (DE)

(57) **Zusammenfassung**

Die Erfindung betrifft eine Grundfeldmagnetanordnung (40) für ein Magnetresonanztomographie-System (1),
welche Grundfeldmagnetanordnung (40) mehrere räumlich voneinander getrennte Grundfeldmagnet-Segmente (44) aufweist, um jeweils ein bestimmungsgemäßes Magnetfeld mit einer definierten Segment-Hauptfeldrichtung (R1) zu erzeugen,
wobei zumindest zwei der Grundfeldmagnet-Segmente (44) so zueinander angeordnet sind, dass die Segment-Hauptfeldrichtungen (R1) ihrer bestimmungsgemäßen Magnetfelder unter einem Ablenkwinkel zueinander verlaufen, so dass die bestimmungsgemäßen Magnetfelder der Grundfeldmagnet-Segmente (44) in einem bestimmungsgemäßen Grundmagnetfeld (B0) resultieren, wobei das Grundmagnetfeld (B0) eine Grundmagnet-Hauptfeldrichtung (R0) mit einem ringförmigen Verlauf aufweist. Die Erfindung betrifft des Weiteren ein Magnetresonanztomographie-System (1), ein Verfahren zur Messung sowie eine Verwendung eines toroidalen Grundmagnetfelds (B0).

## Beschreibung

Die Erfindung betrifft eine Grundfeldmagnetanordnung für ein Magnetresonanztomographie-System, ein entsprechendes Magnetresonanztomographie-System sowie ein Verfahren zur Messung von Rohdaten für eine Magnetresonanztomographieaufnahme.

Seit mehr als vier Jahrzehnten wird das Prinzip der Magnetresonanztomographie ("MR") zur Bildgebung und für andere Messungen verwendet. Trotz dieser langen Zeit und der Bedeutung, welche diese Messmethode mittlerweile erreicht hat, werden bisher nur zwei Magnetdesigns für klinisch eingesetzte MR-Systeme bzw. MR-Scanner verwendet: C-Magnetformen und Solenoide. Der Betrieb dieser Art von MR-Scanner wirft immer noch Probleme für den klinischen MR-Workflow auf.

Die gravierendsten Probleme treten im Hinblick auf die weitreichenden Streumagnetfelder um diese Scanner auf. Um mit dieser Einschränkung umzugehen und Unfälle sowie Schäden zu vermeiden, muss die Krankenhausverwaltung einen streng kontrollierten Bereich innerhalb und in der Nähe der MR-Untersuchungsräume abgrenzen, indem der Zugang von Menschen und Geräten eingeschränkt wird. Schäden können auftreten, wenn metallische oder magnetische Teile von den starken Magneten der MR-Scanner angezogen und in Richtung des Scannervolumens beschleunigt werden.

Ein weiteres Problem besteht darin, dass die MR-Scanner, die ein Solenoid-Magnet-Design verwenden, die Patienten in einem engen Patiententunnel "einschließen", was insbesondere Angstgefühle aufgrund der Enge verursachen kann. Diese Angst kann bei einigen Patienten eine solche Stärke erreichen, dass kein MR-Scan durchgeführt werden kann. Darüber hinaus wird der Zugang des medizinischen Personals zum Patientenkörper aufgrund der Enge des Patiententunnels erheblich eingeschränkt, was ungünstig für die Durchführung von interventionellen oder therapeutischen Verfahren ist, insbesondere im Hinblick auf die Echtzeit-MR-Bildgebung.

Herkömmliche MR-Scanner sind zudem aufgrund ihrer Masse, die insbesondere durch ein Magnetjoch oder Abschirmungen des Magnetfeldes herrührt, nur schwer von einem Ort zu einem anderen zu bewegen. Insbesondere der Transport innerhalb einer Krankenhauseinrichtung oder -abteilung oder mit Lastkraftwagen oder Flugzeugen ist problematisch. Daher ist es schwierig, Patienten an entlegenen Orten oder in geografischen Regionen, die mit Naturkatastrophen oder militärischen Konflikten konfrontiert sind, mit einem Magnetresonanztomographie-System ("MR-System") zu scannen. Stets muss ein Patient zu einem MR-System verbracht werden, da es bisher nicht ohne Probleme möglich ist, ein MR-System schnell an einen Ort zu transportieren.

Üblicherweise verwenden MR-Scanner einen selbstabgeschirmten, supraleitenden Magneten vom Typ eines Solenoiden, um die Stärke des Streumagnetfelds zu verringern, welches aus der Spule des Grundfeldmagneten austritt. Ein aktiv abgeschirmter Grundfeldmagnet ist dabei wesentlich teurer als ein nicht abgeschirmter.

Darüber hinaus reduzieren die Abschirmungsspulen die Effizienz des für Messungen nutzbaren Grundmagnetfeldes in einem Patiententunnel. Aktiv abgeschirmte Magnete haben einen größeren Durchmesser als ungeschirmte Magnete, wobei der Außendurchmesser von typischerweise 145 cm im ungeschirmten Fall auf über 220 cm im geschirmten Fall zunimmt. Dennoch ist im abgeschirmten Fall das magnetische Streufeld nicht vollständig aufgehoben, sondern nur reduziert, so dass im Untersuchungsraum auch bei abgeschirmten Grundfeldmagneten stets ein starkes Streumagnetfeld mit hoher Intensität vorhanden ist. Dieses Streufeld hat immer noch eine solche Stärke, dass die Verwendung von magnetischen Geräten oder medizinischen Instrumenten im Untersuchungsraum nach derzeitigen Regularien nicht zulässig ist.

Alternative Designs für MR-Scanner verwenden einen C-förmigen Magneten. Dies kann entweder ein Permanentmagnet oder ein Elektromagnet sein, der aus zwei Helmholtz-Spulen besteht. Die C-förmigen Magnete haben zwei magnetische Polschuhe, die in ihrem Zwischenraum ein vertikales Grundmagnetfeld erzeugen. Ein analoger Aufbau ist ein Portalmagnet, der mechanisch robuster ist und in einigen Ausführungsformen auch mit supraleitenden Helmholtz-Spulen realisiert werden kann. Die C-Form und die Portalmagnete haben den Vorteil eines offenen Zugangs zum Patientenkörper und reduzieren gleichzeitig klaustrophobische Gefühle der Patienten. Solche ein Aufbau erfordert jedoch eine sehr robuste mechanische Konstruktion, um der enormen magnetischen Anziehungskraft zwischen den beiden gegenüberliegenden Grundfeldmagneten entgegenzuwirken. Um die Ausbreitung von Streumagnetfeldern zu reduzieren, verwenden diese Magnetarchitekturen typischerweise ein Eisenjoch zur Führung der Magnetfeldlinien außerhalb des Abbildungsvolumens. Das Eisenjoch ist eine der kostengünstigsten Abschirmungen. Der Nachteil eines solchen Jochs ist, dass Größe, Gewicht und Volumen des MR-Scanners gravierend erhöht werden.

Bei höheren Feldstärken kann der magnetische Abschirmeffekt eines Eisenjochs aufgrund der Sättigung des Materials und des nichtlinearen Verhaltens reduziert werden. Für Magnete aus supraleitenden Spulen oder Elektromagneten kann eine aktive Abschirmung des Streufeldes durch zusätzliche äußere Spulen mit entgegengesetzter Polarität der Hauptspulen erreicht werden. Diese aktiv abgeschirmte Lösung funktioniert ähnlich wie beim solenoiden Grundfeldmagneten, und weist auch entsprechende Nachteile auf.

Es wurden auch passive Abschirmungen des gesamten MR-Scannerraums mit schweren und dicken Eisenwänden angewendet, um dieses Problem zu umgehen. Dies ist jedoch eine sehr teure Lösung und es wird keinerlei Reduktion das Streumagnetfeld innerhalb des Scannerraumes erreicht.

Es ist eine Aufgabe der vorliegenden Erfindung, eine alternative Grundfeldmagnetanordnung bzw. ein verbessertes Magnetresonanztomographie-System anzugeben, womit die oben beschriebenen Nachteile zumindest reduziert, vorzugsweise vermieden, werden.

Diese Aufgabe wird durch eine Grundfeldmagnetanordnung gemäß Patentanspruch 1, ein Magnetresonanztomographie-System gemäß Patentanspruch 7 sowie durch ein Verfahren gemäß Patentanspruch 12 und eine Verwendung gemäß Patentanspruch 15 gelöst.

Die erfindungsgemäße Grundfeldmagnetanordnung (welche auch als "Grundfeldmagnet-System" bezeichnet werden kann) für ein Magnetresonanztomographie-System weist mehrere räumlich voneinander getrennte (aktive) Grundfeldmagnet-Segmente auf, um (im Betrieb) jeweils ein bestimmungsgemäßes Magnetfeld mit einer definierten Segment-Hauptfeldrichtung zu erzeugen.

Die Grundfeldmagnet-Segmente stellen dabei Teile der Grundfeldmagnetanordnung dar und umfassen mindestens einen Grundfeldmagneten, der durch zumindest eine Magnetspule definiert ist. Es können aber auch mehrere einzelne Grundfeldmagneten bzw. Magnetspulen zu einem Grundfeldmagnet-Segment zusammengeschlossen sein. Auch wenn ein Joch in einem Grundfeldmagneten nicht ausgeschlossen ist, ist es doch zumindest für einige Anwendungen von Vorteil die Grundfeldmagneten so leicht wie möglich zu gestalten, also auf ein Joch zu verzichten. D.h. die Magnetspulen der Grundfeldmagnet-Segmente sind bevorzugt eisenkernfrei, gegebenenfalls mit einem Freiraum im Kernbereich ausgebildet, bzw. die Grundfeldmagnet-Segmente sind bevorzugt eisenjochfrei. Die Grundfeldmagneten können als herkömmliche Elektromagneten oder als supraleitende Elektromagneten ausgestaltet sein. Da die Magnetspulen der Grundfeldmagneten hier im Vordergrund stehen, können die Grundfeldmagnet-Segmente auch insbesondere als "Grundfeldmagnet-Spulensegmente" bezeichnet werden.

Ein bestimmungsgemäßes Magnetfeld (sowie ein bestimmungsgemäßes Grundmagnetfeld) ist dasjenige Magnetfeld, dass sich bei einem bestimmungsgemäßen Betrieb eines Magneten, also wenn ein Strom durch den Magneten fließt, ausbildet. Die Form ist durch das Design des Magneten fest vorgegeben, wobei die Stärke des bestimmungsgemäßen Magnetfelds bei unterschiedlichen Stromstärken in der Stärke skaliert. Da im technischen Bereich der MR-Systeme in der Regel Helmholtz-Spulen verwendet werden, ist das bestimmungsgemäße Magnetfeld eines Grundfeldmagneten meist das eines Solenoiden. Das bestimmungsgemäße Magnetfeld eines Grundfeldmagnet-Segments bzw. der Grundfeldmagnetanordnung kann komplexer sein, zumindest wenn das Grundfeldmagnet-Segment eine andere Form als die einer Helmholtz-Spule hat.

Die Hauptfeldrichtung eines Magnetfelds eines Magneten wird durch denjenigen Vektor angezeigt, der den Magnetfeldverlauf innerhalb des Magneten charakterisiert. Es wird also nicht das Streufeld betrachtet, sondern das Feld, welches im Rahmen der bekannten MR-Systeme primär relevant ist. Bei einem solenoiden Magneten würde die Hauptfeldrichtung senkrecht zu der Stirnfläche des Magneten (Solenoiden) stehen, bei einem toroiden Magneten würde die Hauptfeldrichtung des Magnetfeldes im Inneren des Magneten eine kreisförmige Bahn anzeigen. Bei einem beliebig geformten Magneten gibt die Hauptfeldrichtung den Verlauf des resultierenden Magnetfeldvektors des stärksten Teils des Magnetfeldes (ohne das Streufeld) wieder. Die erwähnte Segment-Hauptfeldrichtung ist die Hauptfeldrichtung eines Magnetfelds eines Grundfeldmagnet-Segments.

In einer erfindungsgemäßen Grundfeldmagnetanordnung sind zumindest zwei der Grundfeldmagnet-Segmente so zueinander angeordnet, dass die Segment-Hauptfeldrichtungen ihrer bestimmungsgemäßen Magnetfelder unter einem Ablenkwinkel zueinander verlaufen. Dieser Ablenkwinkel ist dabei selbstverständlich größer als 0°, da ja sonst keine Ablenkung erreicht werden würde. Die betreffenden Grundfeldmagnet-Segmente sind des Weiteren so zueinander angeordnet, dass die bestimmungsgemäßen Magnetfelder der Grundfeldmagnet-Segmente in einem bestimmungsgemäßen Grundmagnetfeld (der Grundfeldmagnetanordnung) resultieren. Die Grundfeldmagnet-Segmente bilden also im Betrieb gemeinsam das Grundmagnetfeld aus.

Die Grundfeldmagnet-Segmente sind dabei erfindungsgemäß so angeordnet, dass das Grundmagnetfeld eine Grundmagnet-Hauptfeldrichtung mit einem ringförmigen Verlauf aufweist.

Dabei bezeichnet "ringförmig" einen geschlossenen Verlauf, vorzugsweise in einer einzigen Raumebene, bevorzugt in einer Kreisform oder zumindest einer Form mit abgerundeten Ecken. Ein solches ringförmiges Magnetfeld hat ein geringeres Streufeld als das Magnetfeld eines herkömmlichen Solenoiden. Das Streufeld wird kleiner, umso mehr Grundfeldmagnet-Segmente verwendet werden bzw. je ausgedehnter die Grundfeldmagnet-Segmente in Richtung des Magnetfeldverlaufs sind. Verglichen mit einem C-Magneten ist das Gesamtgewicht der erfindungsgemäßen Grundfeldmagnetanordnung um ein vielfaches kleiner, insbesondere da hier auf ein Eisenjoch verzichtet werden kann, dass in C-Magneten üblicherweise benötigt wird, um das Streufeld zu reduzieren.

Ein erfindungsgemäßes Magnetresonanztomographie-System umfasst, in der Regel neben den anderen üblichen Komponenten wie insbesondere einem Gradientensystem, einem HF-Sendesystem, einem HF-Empfangssystem, einer geeigneten Steuereinrichtung etc., eine erfindungsgemäße Grundfeldmagnetanordnung.

Ein erfindungsgemäßes Verfahren zur Messung von Rohdaten für eine Magnetresonanztomographieaufnahme, insbesondere im Rahmen der Magnetresonanztomographie-Bildgebung umfasst die folgenden Schritte:
- Positionieren eines Objekts
   Während dieses Schritts wird ein Patient bzw. ein Proband oder auch ein unbelebtes Objekt wie z.B. ein Phantom in einem Messplatz eines erfindungsgemäßen Magnetresonanztomographie-Systems positioniert.
- Erzeugen eines Grundmagnetfelds
   Dieses Grundmagnetfeld wird mit der erfindungsgemäßen Grundfeldmagnetanordnung des Magnetresonanztomographie-Systems erzeugt.
- Messung der Rohdaten
   In diesem Schritt wird wie üblich zumindest ein interessierender Bereich (RoI für engl.: "Region of Interest") des Objekts mit geeigneten HF-Signalen bzw. einer HF-Pulssequenz angeregt (MR-Anregung) und die dadurch induzierten MR-Signale aus dem RoI als Rohdaten aufgenommen (d.h. die Rohdaten werden akquiriert), die dann mit den verschiedensten Verfahren weiterverarbeitet werden können. Hierzu können auch dem Fachmann bekannte Verfahren eingesetzt werden. Diese Rohdaten dienen nach entsprechender Verarbeitung bevorzugt zur Bildgebung, d.h. zur Erzeugung von Magnetresonanzbilddaten, können aber auch durchaus anderen Ergebnissen dienen. Während der Messung, also der Anregung und/oder Rohdatenakquisition, kann auch hier typischerweise eine Ortskodierung durch zusätzliche Gradientenfelder erfolgen.

Teil der Erfindung ist auch eine Verwendung eines toroidalen Grundmagnetfelds für eine Messung im Rahmen der Magnetresonanztomographie und/oder in einem Resonanztomographie-System. Vorzugsweise findet dabei eine Messung an mehr als einem Messplatz innerhalb eines gemeinsamen toroidalen Grundmagnetfelds statt. Als ein "torodiales" Magnetfeld, also ein Magnetfeld, welches ähnlich zu dem Magnetfeld eines Toroiden ist, werden neben (im Wesentlichen kreisförmigen) toroidförmigen Magnetfeldern selbst insbesondere auch andere in sich geschlossene Magnetfelder angesehen, welche die Form einer Ellipse, eines Rechtecks mit abgerundeten Ecken oder eine Form aus Kreissegmenten und "eingefügten" geraden Passagen haben (insbesondere die Form einer einfachen Rennbahn mit 180° Kehren und zwei gegenläufigen geraden Strecken). Insbesondere befasst sich die Verwendung mit der Verwendung einer erfindungsgemäßen Grundfeldmagnetanordnung wie vorangehend beschrieben.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Gemäß einer bevorzugten Ausführungsform umfasst die Grundfeldmagnetanordnung mindestens drei Grundfeldmagnet-Segmente (bevorzugt mindestens vier, weiter bevorzugt mindestens sechs oder insbesondere bevorzugt mindestens acht), welche so angeordnet sind, dass die Grundmagnet-Hauptfeldrichtung die Form eines ebenen Rings, bevorzugt im Wesentlichen eines Toroids oder eine toroid-ähnliche Form, insbesondere die Form eines Toroids mit eingefügten geraden Passagen, aufweist (siehe oben). Dies bedeutet, dass die Segment-Hauptfeldrichtungen der Magnetfelder der Grundfeldmagnet-Segmente alle in einer einzigen gemeinsamen Raumebene liegen.

Gemäß einer bevorzugten Ausführungsform der Grundfeldmagnetanordnung beträgt der Ablenkwinkel der Segment-Hauptfeldrichtungen zwischen zumindest zwei benachbarten Grundfeldmagnet-Segmenten mindestens 5°, bevorzugt mindestens 30°, besonders bevorzugt mindestens 45°. Dies heißt, dass die Grundfeldmagnet-Segmente (bezüglich ihrer Segment-Hauptfeldrichtung) entsprechend zueinander verkippt angeordnet sind. Diesbezüglich ist bevorzugt, dass Grundfeldmagnet-Segmente mit einer ihrer Seitenwände einander zugewandt sind und die Wickel- bzw. Spulenebenen benachbarter Grundfeldmagnet-Segmenten zueinander verkippt sind. Die Seitenwände sind hier die seitlichen Wände in denen Stromleiter der Magnetwicklungen verlaufen. Man könnte die Seitenwände auch als Kanten bezeichnen. Als Seitenwände sind also nicht die Stirnseiten eines Magneten zu verstehen (aus denen das Magnetfeld austritt und die z. B. im Wesentlichen parallel zu einer Wickelebene einer Magnetspule des Grundfeldmagnet-Segments liegen), sondern die Seiten in einer Richtung quer zur Segment-Hauptfeldrichtung des Grundfeldmagnet-Segments.

Gemäß einer bevorzugten Ausführungsform umfasst die Grundfeldmagnetanordnung zumindest eine Gruppe von Grundfeldmagnet-Segmenten, die sternförmig um zumindest eine Raumachse herum angeordnet sind, wobei jeweils eine Seitenwand bzw. Kanten des jeweiligen Grundfeldmagnet-Segments zu dieser Mittelachse zeigt. Diese Anordnung ist bevorzugt drehsymmetrisch, wobei besonders bevorzugt bei N Grundfeldmagnet-Segmenten (in einer Gruppe) eine Drehsymmetrie von 360°/N vorliegt. Bei sechs Grundfeldmagnet-Segmenten sähe die Grundfeldmagnetanordnung z.B. aus wie ein sechszackiger Stern. Eine Sternform kann aber auch eine teilweise regelmäßige Anordnung von Grundfeldmagnet-Segmenten umfassen, z.B. dass die Grundfeldmagnet-Segmente alle regelmäßig innerhalb eines Halbkreises angeordnet sind. Bevorzugt ist auch eine Anordnung von mehreren dieser teilweise regelmäßigen Sternformen um mehrere Mittelachsen bzw. Raumachsen, z.B. zwei halbkreisförmige Anordnungen, die sich etwas beabstandet gegenüberliegen, um insgesamt z.B. das bereits erwähnte Grundmagnetfeld in der Form eines Toroids mit eingefügten geraden Passagen zu erzeugen.

Gemäß einer bevorzugten Ausführungsform umfasst die Grundfeldmagnetanordnung ein Grundfeldmagnet-Segment oder eine Gruppe von Grundfeldmagnet-Segmenten, welche aufgebaut ist, um die Grundmagnet-Hauptfeldrichtung des bestimmungsgemäßen Grundmagnetfelds um einen Gesamt-Ablenkwinkel von mindestens 60°, bevorzugt von mindestens 90°, weiter bevorzugt von mindestens 180° abzulenken. Bevorzugt verläuft dabei die Segment-Hauptfeldrichtung des Magnetfeldes dieses Grundfeldmagnet-Segments bzw. die resultierende Segment-Hauptfeldrichtung des resultierenden Magnetfeldes dieser Gruppe (also die Segmentgruppen-Hauptfeldrichtung) in einem Bogen, der eine Ablenkung um besagte Winkel darstellt. Dieses Grundfeldmagnet-Segment bzw. diese eine Gruppe von Grundfeldmagnet-Segmenten kann dazu verwendet werden, das Grundmagnetfeld gezielt zu führen.

Insbesondere wenn wie oben beschrieben Grundfeldmagnet-Segmente sternförmig oder in ähnlicher Weise um eine Mittelachse angeordnet werden, um einen ringförmig geschlossenen Verlauf der Grundmagnet-Hauptfeldrichtung des Grundmagnetfelds zu erreichen, und die einzelnen Grundfeldmagnet-Segmente so aufgebaut sind, dass sie ein Magnetfeld erzeugen, welches in einer Raumrichtung senkrecht zu ihrer Segment-Hauptfeldrichtung im Wesentlichen homogen sind (also wenn die Grundfeldmagnet-Segmente beispielsweise in Form von Helmholtz-Spulen aufgebaut sind), kann dies zu einem Grundmagnetfeld führen, dessen Feldstärke in radialer Richtung (ausgehend von der Mittelachse) inhomogen ist, insofern dass es (stetig) abnimmt. Dies liegt daran, dass der Abstand zwischen den einzelnen Grundfeldmagnet-Segmenten nah an der Raumachse enger ist als in einem radial größeren Abstand von der Mittelachse. D.h. die Dichte der Feldlinien des resultierenden Grundmagnetfelds nimmt radial nach außen hin ab.

Insbesondere um dies auszugleichen und ein auch in radialer Richtung möglichst homogenes Grundmagnetfeld zu erreichen, kann in einer bevorzugten Ausführungsform der Grundfeldmagnetanordnung ein Grundfeldmagnet-Segment (vorzugsweise jedes der Grundfeldmagnet-Segmente) eine Spulenwicklung aufweisen, die ein bestimmungsgemäßes Magnetfeld erzeugt, das zu einer Seite des Grundfeldmagnet-Segments, d.h. in einer Richtung quer zur Segment-Hauptfeldrichtung des Grundfeldmagnet-Segments, hin stärker wird. Dazu ist die Spulenwicklung bevorzugt so gestaltet, dass der Durchmesser einer Wicklung in zumindest einer Raumrichtung, verglichen mit ihrer benachbarten Wicklung, abnimmt und ihr Mittelpunkt dichter an einer Seite des ringförmigen Grundmagnetfelds liegt. Das Magnetfeld dieses einzelnen Grundfeldmagnet-Segments wird also bevorzugt zum Außenrand der Grundfeldmagnetanordnung (z.B. zum Außenrand der Kreisform, insbesondere Toroidform) hin stärker, um die ansonsten auftretende radiale Inhomogenität des insgesamt resultierenden Grundmagnetfelds möglichst gut auszugleichen. Man kann bezüglich der Grundfeldmagnet-Segmente auch sagen, dass die Wicklungen nach und nach zur äußeren Seitenwand eines Grundfeldmagnet-Segments hin tendieren, also der Außenseite der Ringform des Grundmagnetfeldes. Die genaue Art bzw. Topographie der Wicklung wird hierbei bevorzugt entsprechend an die Anordnung der Grundfeldmagnet-Segmente zueinander angepasst, um einen geeigneten Ausgleich der Inhomogenität zu erreichen.
Ein bevorzugtes Magnetresonanztomographie-System umfasst eine Mehrzahl von Messplätzen (mindestens zwei) innerhalb des bestimmungsgemäßen (gemeinsamen) Grundmagnetfelds. Diese Messplätze sind dabei vorzugsweise zwischen jeweils zwei Grundfeldmagnet-Segmenten und/oder in einem Patiententunnel innerhalb eines Grundfeldmagnet-Segments angeordnet.

Bevorzugt umfasst das Magnetresonanztomographie-System hierzu entsprechend eine Mehrzahl von Messeinrichtungen (mindestens zwei), wobei jede der Messeinrichtungen zur Durchführung einer Messung im Rahmen der Magnetresonanztomographie an einem der vorgenannten Messplätzen ausgelegt ist. Bevorzugt weist die Messeinrichtung hierzu jeweils zumindest ein HF-Sendesystem zur Applikation einer HF-Pulssequenz (also zur Ausendung der HF-Pulse zur MR-Anregung) und ein HF-Empfangssystem zum Auslesen der dadurch indizierten MR-Signale auf. Die Messeinrichtung kann selbstverständlich noch weitere Komponenten aufweisen, wie z.B. eine Gradientenspule. Dies wird in der weiteren Beschreibung noch näher ausgeführt.

Insbesondere könnten auch das HF-Sendesystem und das HF-Empfangssystem zumindest teilweise kombiniert sein, zum Beispiel indem sie eine gemeinsame HF-Sende-/Empfangsspule (Antenne) nutzen, wie dies auch in herkömmlichen Magnetresonanz-Tomographiesystemen der Fall ist. Beispielsweise kann eine Messeinrichtung hierzu eine am Objekt, insbesondere Patienten, anzubringende Lokalspule aufweisen, welche als HF-Sendespule und/oder HF-Empfangsspule genutzt werden kann.

Verschiedene geeignete Messeinrichtungen, die mit gegebenenfalls geringen Abwandlungen an den verschiedenen Messplätzen genutzt werden könnten, sind im Rahmen der Magnetresonanztomographie im Grunde bekannt, wobei im Stand der Technik in der Regel zur Zeit nur eine einzige Messeinrichtung pro MR-System genutzt wird, bzw. nur ein Messplatz vorhanden ist, an dem wahlweise eine Messeinrichtung genutzt wird, auf jeden Fall aber immer nur ein Patient gleichzeitig bedient werden kann.

Im Rahmen der Erfindung ist bevorzugt, dass die Messeinrichtungen voneinander unabhängig sind, insbesondere räumlich und/oder messtechnisch. Sie können allerdings von einer gemeinsamen Steuereinrichtung koordiniert angesteuert werden, wobei die Steuereinrichtung auch die MR-Signale bzw. Rohdaten empfangen und verarbeiten kann. Ebenso kann jede Messeinrichtung aber auch eine eigene Steuereinrichtung aufweisen.

Gemäß einem bevorzugten Verfahren werden entsprechend an zumindest zwei Messplätzen gleichzeitig Magnetresonanztomographieaufnahmen durchgeführt. Dies bedeutet, dass gleichzeitig zwei Objekte an diesen Messplätzen untersucht werden oder ggf. andere Messungen durchgeführt werden. Dafür ist jedoch nicht zwingend erforderlich, dass gleichzeitig eine Rohdatenmessung, also HF-Anregung und/oder Rohdatenakquise erfolgen muss, diese kann auch in geeigneter Weise koordiniert sein. Beispielsweise könnte wechselweise gemessen werden, um eine wechselseitige Störung zu vermeiden, d.h. das immer an einem der Messplätze eine Messung durchgeführt wird (also HF-Pulse ausgesendet und Rohdaten akquiriert werden) während an einem anderen der Messplätze gerade ein Patient positioniert wird, eine Verschiebung der Position des Patienten (z.B. der Vorschub einer Patientenliege) erfolgt oder ein Kontrastmittel verabreicht wird. Bekanntermaßen blockiert ja auch die Vorbereitungs- und Nachbereitungszeit des Untersuchungsablaufs das Magnetresonanztomographie-System über einen nicht unerheblichen Zeitraum, der so effektiver genutzt werden könnte.

Insofern ist also eine "gleichzeitige Durchführung von Magnetresonanztomographieaufnahmen" so zu verstehen, dass zu einer Magnetresonanztomographieaufnahme (nachfolgend auch einfach "Bildgebung" genannt) der Zeitraum gehört, in den alle Maßnahmen fallen, die zu einer typischen MR-Messung gehören: Positionierung des Patienten, ggf. Applikation eines Kontrastmittels, Applikation einer Pulssequenz, Messung der MR-Signale, und ggf. Rekonstruktion eines Bildes, entlassen des Patienten aus dem Untersuchungsraum etc., also alle Vorgänge, die das MR-System belegen. Die Rohdatenmessung umfasst hierbei - als ein Teil der Bildgebung - nur die Applikation einer Pulssequenz bzw. eine HF-Anregung und eine Messung der dadurch induzierten MR-Signale. Die Erfassung der MR-Signale selbst wird auch als "Rohdatenakquise" bezeichnet.

Eine gleichzeitige Durchführung von Magnetresonanztomographieaufnahmen bedeutet also, dass sich die Zeiträume von Bildgebungen an verschiedenen Messplätzen zeitlich überdecken bzw. überlappen können. Dabei kann es von Vorteil sein, wenn innerhalb diese gemeinsam beanspruchten Zeitraums aktive Prozesse, die das Magnetfeld oder eine andere Messung beeinflussen können, getrennt voneinander durchgeführt werden. Passive Prozesse, also Prozesse, die keinen Einfluss auf eine andere Messung haben, wie z.B. ein Vorschub eines Patiententisches oder die Gabe eines Kontrastmittels, können wie erwähnt in der Regel durchaus gleichzeitig erfolgen. Das schließt aber nicht aus, dass je nach Aufbau und Anordnung der Messplätze zueinander auch aktive Prozesse oder die Messung von Rohdaten an verschiedenen Messplätzen gleichzeitig erfolgen kann.

Gegebenenfalls könnte beispielsweise bei zwei parallelen Messungen mit unterschiedlichen Echozeiten die Anregungen, also die Applikation von Signalen an einem Messplatz stets dann erfolgen, wenn auf dem anderen Messplatz keine Signale appliziert werden. Die eigentliche Datennahme (Rohdatenakquise) kann aber durchaus gleichzeitig erfolgen. Insbesondere kann auf diese Weise die Aufnahme von einzelnen Schichten bei zwei parallelen Messungen geschachtelt bzw. abwechselnd erfolgen. In diesem Rahmen ist eine zentral koordinierte Steuerung sehr von Vorteil, wie sie bereits vorangehend beschrieben worden ist. Es ist insbesondere in dieser Hinsicht besonders bevorzugt, einen Abgleich von Gradienten der einzelnen Messungen durchzuführen, so dass diese sich z.B. nicht gegenseitig beeinflussen.

Ein bevorzugtes Magnetresonanztomographie-System weist eine Steuereinrichtung zur Ansteuerungen aller Komponenten der Grundfeldmagnetanordnung und der Messeinrichtung(en) dieses Magnetresonanztomographie-Systems auf, wobei diese Steuereinrichtung vorzugsweise auch so ausgebildet sein kann, dass die Messungen an verschiedenen Messplätzen koordiniert ablaufen, wie dies vorangehend erläutert wurde.

Ein Teil der Komponenten der Steuereinrichtung, insbesondere Komponenten die für eine Koordinierung der Messungen sorgen, können ganz oder teilweise in Form von Softwaremodulen in einem Prozessor einer entsprechenden Steuereinrichtung eines MR-Systems realisiert werden. Eine weitgehend softwaremäßige Realisierung solcher Komponenten hat den Vorteil, dass auch schon bisher verwendete Steuereinrichtungen auf einfache Weise durch ein Software-Update nachgerüstet werden können, um auf die erfindungsgemäße Weise zu arbeiten.

Die Steuereinrichtung kann auch mehrere Teil-Steuereinrichtungen umfassen, die zum Beispiel verschiedenen Messplätzen und/oder Messeinrichtungen zugeordnet sind und sich vorzugsweise zur Koordinierung untereinander verständigen können und/oder durch einen übergeordnete Master-Steuereinheit koordiniert werden.

Vorzugsweise kann jede Messeinrichtung auch ein eigenes Gradientensystem und/oder Shimspulen-System aufweisen, bzw. es kann jedem Messplatz im Magnetresonanztomographie-System ein eigenes Gradientensystem und/oder Shimspulen-System zugeordnet sein.

So kann bevorzugt, wie oben bereits erwähnt, die Grundfeldmagneanordnung so ausgestaltet sein, dass an einem Messplatz das Grundmagnetfeld möglichst homogen ist. Um die Homogenität noch weiter zu verbessern, kann das Shimspulen-System eingesetzt werden. Hierzu kann auch auf Vorgehensweisen bzw. Prinzipien aus dem Stand der Technik an herkömmlichen Magnetresonanztomographie-Systemen zurückgegriffen werden, die gegebenenfalls in geeigneter Weise angepasst werden. Mit dem Gradientensystem können dann die gewünschten Gradientenfelder für die Messung angelegt werden.

Bei anderen bevorzugten Ausführungsformen kann aber auch eine vorliegende bekannte bzw. klar definierte Inhomogenität des ringförmigen Grundmagnetfeldes gezielt genutzt werden, z.B. zur Ortscodierung der Messdaten bzw. Rohdaten.

Hierzu kann insbesondere ausgenutzt werden, dass bei einem ringförmigen Verlauf eines Magnetfeldes, wie oben erwähnt, ohne geeignete Gegenmaßnahmen in der Regel zum Mittelpunkt des Rings hin ein stärkeres Magnetfeld herrscht als am Rand. In der Regel ist die Abnahme der Feldstärke antiproportional zum Abstand vom Mittelpunkt des Rings. Da zur Ortscodierung im Rahmen von MR-Messungen in der Regel eine Inhomogenität des Magnetfeldes genutzt wird, welches aber bisher üblicherweise durch das Gradientenspulensystem eingestellt wird, kann eine durch den Aufbau der Grundfeldmagnetanordnung auftretende Inhomogenität des Grundmagnetfelds vorteilhaft genutzt werden. Zumindest ist es nicht unbedingt erforderlich, in der Richtung der Inhomogenität ein Gradientenfeld zu applizieren, wie es bisher im Stand der Technik getan wird.

Es sei ergänzend erwähnt, dass auch Kombinationen der verschiedenen Varianten möglich sind, also dass beispielsweise nur in einigen Raumrichtung die Inhomogenität des Grundmagnetfelds ausgenutzt wird und in einigen Raumrichtungen zusätzliche Gradientenfelder eingesetzt werden oder dass dies auch von Messeplatz zu Messeplatz unterschiedlich gehandhabt wird.

Vorzugsweise sind zumindest zwei Messplätze in Bereichen mit einer gegenläufigen Grundmagnet-Hauptfeldrichtung angeordnet. Bei einer toroidförmigen Grundmagnet-Hauptfeldrichtung würden diese Messplätze an entgegengesetzten Seiten des Grundmagnetfeldes liegen. Die Messplätze sind bevorzugt nebeneinander angeordnet und liegen besonders bevorzugt auf einer gemeinsamen Ebene.

Wie bereits oben erwähnt kann die Grundfeldmagnetanordnung eines erfindungsgemäßen Magnetresonanztomographie-Systems bevorzugt ein Grundfeldmagnet-Segment oder eine Gruppe von Grundfeldmagnet-Segmenten aufweisen, welche aufgebaut ist, um die Grundmagnet-Hauptfeldrichtung des bestimmungsgemäßen Grundmagnetfelds um einen Gesamt-Ablenkwinkel von mindestens 60°, bevorzugt von mindestens 90°, weiter bevorzugt von mindestens 180° abzulenken. Eine solche Grundfeldmagnetanordnung kann insbesondere auch für die vorgenannte bevorzugte Ausführungsform verwendet werden, um die Grundmagnet-Hauptfeldrichtung von einem Messplatz zu einem anderen Messplatz um 180° abzulenken, so dass die Grundmagnet-Hauptfeldrichtung an diesen Messplätzen gegenläufig ist.

Es ist bevorzugt, dass ein Grundfeldmagnet-Segment oder eine Gruppe von Grundfeldmagnet-Segmenten unter oder über zumindest einem Messplatz oder an zumindest einer Seite eines Messplatzes angeordnet ist, wobei vorzugsweise dieses Grundfeldmagnet-Segment oder die Gruppe von Grundfeldmagnet-Segmenten an zwei unterschiedliche Messplätze angrenzt.

In einem bevorzugten Magnetresonanztomographie-System mit mehreren Messplätzen kann ein Grundfeldmagnet-Segment (z.B. ein Grundfeldmagnet) auch bevorzugt als Wand ausgebildet und/oder in einer Wand zwischen zwei Messplätzen angeordnet sein.

Zu einer besonders bevorzugten Variante zählt hier eine Grundfeldmagnetanordnung mit einer Gruppe von in einem Halbkreis regelmäßig angeordneten Grundfeldmagnet-Segmenten, wobei diese Gruppe das Grundmagnetfeld um 180° ablenkt, und einem weiteren Grundfeldmagnet-Segment, welches mittig senkrecht zu diesem Halbkreis angeordnet ist (als quasi auf diesem steht) und sich zwischen den beiden Messplätzen befinden kann. Dies wird später noch an einem bevorzugten Ausführungsbeispiel genauer erläutert.

Diese Erfindung lässt im Vergleich mit der traditionellen Magnetresonanztomographie, die auf homogene Grundmagnetfelder mit rechtwinkligen und parallelen Feldlinien basieren, Variationen in der Technik der Bildaufnahme, insbesondere der Signalcodierung, und der Bildrekonstruktion zu. Hier folgen die Signalcodierung und die Bildrekonstruktion den "Isofrequenzflächen", also Flächen mit gleicher Frequenz der aufgenommenen Bereiche. Diese Isofrequenzflächen sind in einem toroidalen Magnetfeld gekrümmt und folgen Flächen mit jeweils gleicher Magnetfeldstärke.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen schematisch:
Figur 1 eine Darstellung eines Magnetresonanztomographie-Systems gemäß dem Stand der Technik,
Figur 2 eine vereinfachte Darstellung eines Ausführungsbeispiels für ein Magnetresonanztomographie-System mit einer erfindungsgemäßen Grundfeldmagnetanordnung,
Figur 3 eine schematische Darstellung einer Grundfeldmagnetanordnung, wie sie in dem Ausführungsbeispiel gemäß Figur 2 genutzt werden kann,
Figur 4 eine etwas detailliertere Darstellung einer Grundfeldmagnetanordnung, wie sie in dem Ausführungsbeispiel gemäß Figur 2 genutzt werden kann,
Figur 5 ein weiteres Ausführungsbeispiel für ein Magnetresonanztomographie-System mit einer erfindungsgemäßen Grundfeldmagnetanordnung,
Figur 6 ein weiteres Ausführungsbeispiel für ein Magnetresonanztomographie-System mit einer erfindungsgemäßen Grundfeldmagnetanordnung,
Figur 7 ein weiteres Ausführungsbeispiel für ein Magnetresonanztomographie-System mit einer erfindungsgemäßen Grundfeldmagnetanordnung,
Figur 8 eine schematische Darstellung einer bevorzugten Spulenwicklung, wie sie beispielsweise in den Grundfeldmagnetanordnungen nach den Figuren 1 bis 7 genutzt werden kann,
Figur 9 eine detaillierte Darstellung einer Grundfeldmagnetanordnung ähnlich der in Figur 2, jedoch nun mit einer Spulenwicklung wie in Figur 8,

In den folgenden Figuren sind nur für die Erfindung wesentliche oder zu ihrem Verständnis hilfreiche Elemente eingezeichnet.

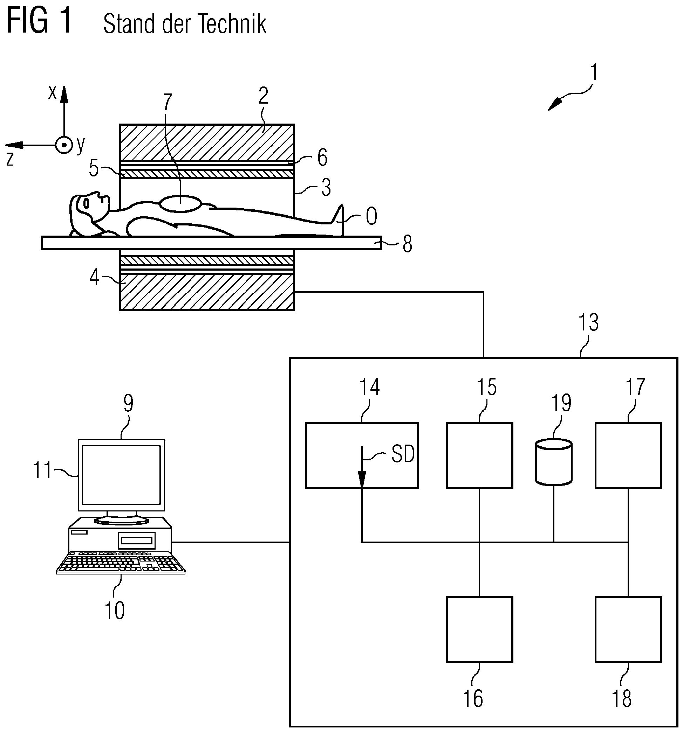

In Figur 1 ist grob schematisch ein Magnetresonanztomographie-System 1 dargestellt. Es umfasst zum einen den eigentlichen Magnetresonanzscanner 2 mit einem Messplatz 3 bzw. Untersuchungsraum 3, hier einem klassischen Patiententunnel 3, in dem auf einer Liege 8 ein Patient O oder Proband, d.h. das Untersuchungsobjekt O positioniert ist. In der Regel wird aber nicht der ganze Patient O gescannt, sondern nur eine interessierende Regionen innerhalb des Patienten O, d.h. nur aus diesem Bereich werden Rohdaten gemessen.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennensystem 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten O bzw. Probanden anzuordnenden Lokalspulen besteht. Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Grundfeldmagnetsystem 4 ist hier in üblicher Weise so ausgebildet, dass es ein Grundmagnetfeld in Längsrichtung des Patienten erzeugt, d.h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können. Zudem kann der Magnetresonanzscanner 2 (nicht dargestellte) Shimspulen aufweisen, die in üblicher Weise ausgebildet sein können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz oder einer Abfolge von mehreren Pulssequenzen zur Aufnahme mehrerer Schichten in einem interessierenden Volumenbereich des Untersuchungsobjekts O innerhalb einer Messsitzung gesteuert. Eine solche Pulssequenz kann beispielsweise innerhalb eines Mess- oder Steuerprotokolls vorgegeben und parametrisiert sein. Üblicherweise sind verschiedene Steuerprotokolle für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden. Im vorliegenden Fall enthält die Steuereinrichtung 13 Pulssequenzen zur Messung der Rohdaten, also zur Anregung der MR-Signale für die Akquisition der Rohdaten.

Zur Ausgabe der einzelnen HF-Pulse einer Pulssequenz weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend der vorgegebenen Pulssequenz die Gradientenpulse passend zu schalten, weist die Steuereinrichtung 13 eine Gradientensystemschnittstelle 16 auf. Die Sequenzsteuereinheit 14 kommuniziert in geeigneter Weise, z. B. durch Aussendung von Sequenzsteuerdaten SD, mit der Hochfrequenzsendeeinrichtung 15 und der Gradientensystemschnittstelle 16 zur Ausführung der Pulssequenz.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz vorgegebenen Auslesefenster koordiniert mittels des HF-Empfangsantennensystems 7 Magnetresonanz-Signale zu empfangen und so die Rohdaten zu akquirieren.

Eine Rekonstruktionseinheit 18 übernimmt hier die akquirierten Rohdaten und rekonstruiert daraus Magnetresonanz-Bilddaten. Auch diese Rekonstruktion erfolgt in der Regel auf Basis von Parametern, die in dem jeweiligen Mess- oder Steuerprotokoll vorgegeben sein können. Diese Bilddaten können dann beispielsweise in einem Speicher 19 hinterlegt werden.

Wie im Detail durch ein Einstrahlen von HF-Pulsen und das Schalten von Gradientenpulsen geeignete Rohdaten akquiriert und daraus MR-Bilder oder Parameter-Karten etc. rekonstruiert werden können, ist dem Fachmann grundsätzlich bekannt und wird daher hier nicht näher erläutert.

Eine Bedienung der zentralen Steuereinrichtung 13 kann über ein Terminal 11 mit einer Eingabeeinheit 10 und einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanztomographie-Bilder angezeigt werden, und mittels der Eingabeeinheit 10, gegebenenfalls in Kombination mit der Anzeigeeinheit 9, können Messungen geplant und gestartet und insbesondere Steuerprotokolle ausgewählt und gegebenenfalls modifiziert werden.

Solch ein Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten bzw. Parameterkarten, aber auch weitere Daten, wie beispielsweise patientenrelevante Daten oder Steuerprotokolle, austauschen zu können.

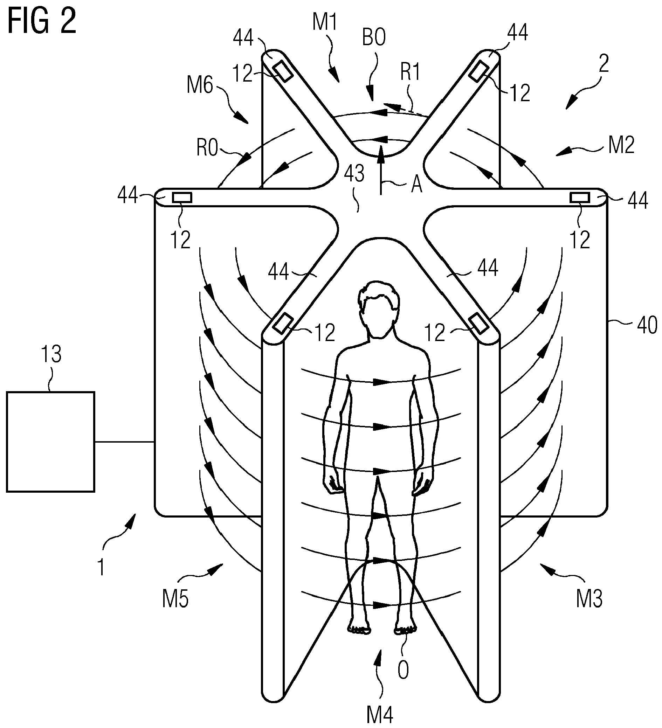

Figur 2 zeigt ein Ausführungsbeispiel für ein Magnetresonanztomographie-System 1 mit einer erfindungsgemäßen Grundfeldmagnetanordnung 40.

Dargestellt ist hier ein Magnetresonanzscanner 2, dessen Funktion von einer Steuereinrichtung 13 gesteuert werden kann. Die Steuereinrichtung 13 kann dabei im Prinzip in ähnlicher Weise aufgebaut sein und die gleichen Komponenten aufweisen, wie die Steuereinrichtung 13 in einem herkömmlichen MR-System gemäß Figur 1. Ebenso kann diese auch ein geeignetes Terminal oder dergleichen aufweisen (was hier aber nicht dargestellt ist).

Die Grundfeldmagnetanordnung 40 des Magnetresonanzscanners 2 umfasst hier sechs (hier identische) Grundfeldmagnet-Segmente 44, die in dieser Ausführungsform sternförmig um eine Mittelachse A mit einer Drehsymmetrie von 60° angeordnet sind. Das durch Pfeile angedeutete Grundmagnetfeld B0 hat eine Grundfeld-Hauptrichtung R0, die in Form eines Kreises bzw. eines toroiden Magnetfeldes verläuft.

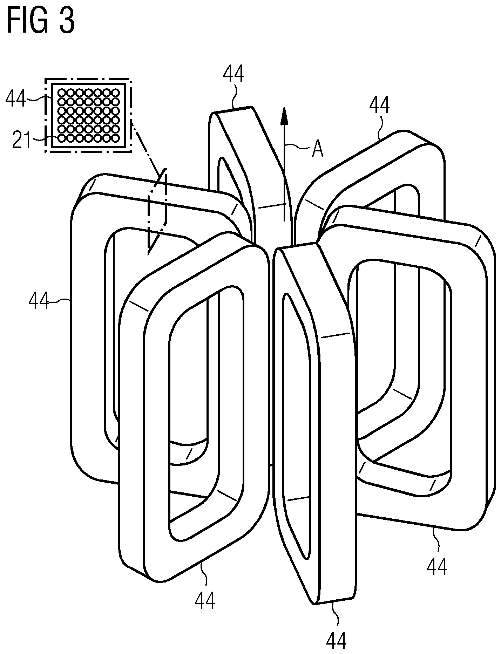

Figur 3 zeigt hierzu eine detaillierte schematische Darstellung der einzelnen Grundfeldmagnet-Segmente 44 der sternförmigen Grundfeldmagnetanordnung 40 in Figur 2.

Zu sehen sind hier sechs Helmholtz-Spulen als Grundfeldmagnet-Segmente 44 der Grundfeldmagnetanordnung 40. Sie sind mit einer Kante ihrer Spulenebene bzw. Wickelebene zur Mittelachse A hin ausgerichtet. Jedes einzelne Grundfeldmagnet-Segment 44 hat ein bestimmungsgemäßes Magnetfeld, welches dem eines (relativ kurzen) Solenoiden entspricht, d. h. die Segment-Hauptfeldrichtung R1 (in der Darstellung in Figur 2 nur bei einem der hinteren beiden Grundfeldmagnet-Segmente 44 gezeigt) des Magnetfelds, welches von einem einzelnen Grundfeldmagnet-Segment 44 erzeugt wird, würden jeweils senkrecht zur Stirnfläche des betreffenden Grundfeldmagnet-Segments 44 stehen. Gemeinsam resultieren die einzelnen Magnetfelder der Grundfeldmagnet-Segmente 44 in dem in Figur 2 angezeigten Grundmagnetfeld B0 mit einer toroiden Grundmagnet-Hauptfeldrichtung RO, wobei die Segment-Hauptfeldrichtung R1 im Mittelpunkt der einzelnen Grundfeldmagnet-Segmente 44 jeweils "tangential" zur kreisförmigen Grundmagnet-Hauptfeldrichtung R0 steht. Das Grundmagnetfeld B0 nimmt in radialer Richtung nach außen hin ab, ist jedoch in der Höhe homogen.

Die Grundfeldmagnet-Segmente 44 der Grundfeldmagnetanordnung 40 sind so miteinander verschaltet, dass ein Gleichstrom von einem Grundfeldmagnet-Segment 44 in das nächste fließt, wobei die Stromrichtung durch die Magnetwicklungen stets dieselbe ist und sich durch den Stromfluss insgesamt das kreisförmiges Magnetfeld B0 ausbildet.

Ein signifikanter Vorteil einer solchen symmetrischen Anordnung ist die strukturelle Stabilität bei eingeschaltetem Grundmagnetfeld B0. Die magnetischen Kräfte zwischen den einzelnen Grundfeldmagnet-Segmenten 44 heben sich in Richtung der Grundmagnet-Hauptfeldrichtung R0 gegenseitig auf. Jedes Grundfeldmagnet-Segment 44 wird von seinen beiden Nachbarn mit jeweils der gleichen Kraft angezogen. Die resultierenden Kräfte wirken nach innen zur Säule 43 hin und können dort sehr gut durch entsprechende strukturelle Verstärkungen kompensiert werden.

In der linken oberen Ecke der Figur 3 ist vergrößert ein Schnitt durch ein Grundfeldmagnet-Segment 44 gezeigt. Zu sehen ist eine regelmäßige Anordnung von Stromleitern 21, die hier als Drähte eingezeichnet sind, jedoch durchaus einen komplexen Aufbau haben können, z.B. hohl sein können, um ein Kühlmittel hindurchzuleiten.

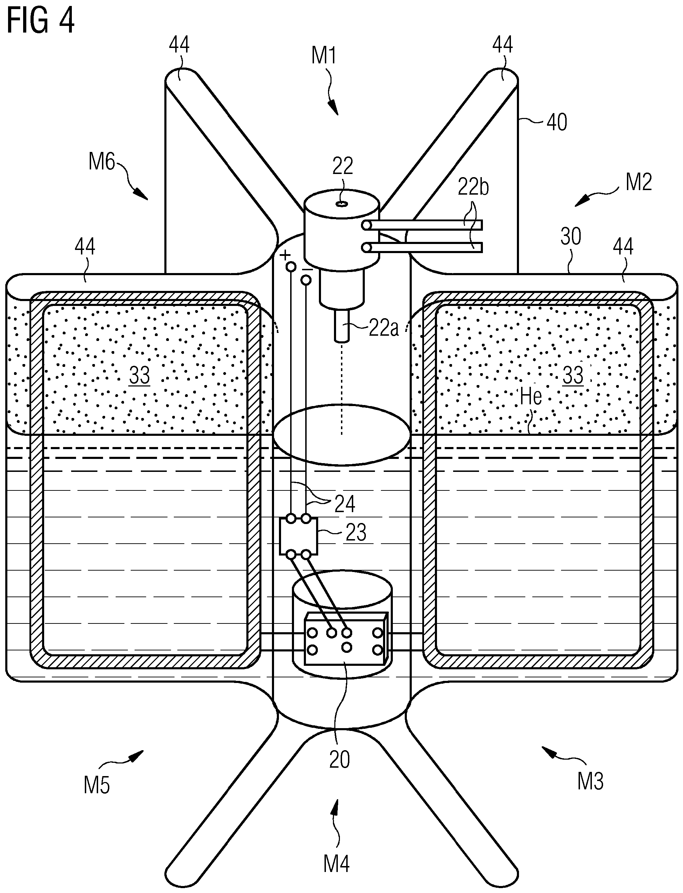

Ein solches Magnetresonanztomographie-System 1 mit einer Grundfeldmagnetanordnung 40 gemäß den Figuren 2 und 3 erlaubt Messungen an sechs verschiedenen Messplätzen M1, M2, M3, M4, M5, M6 (siehe Figur 2), wobei in dem dargestellten Beispiel gerade an Messplatz M4 eine Messung eines Objektes O stattfindet, wobei ein Patient hier aufrecht an senkrechten Wänden der Grundfeldmagnetanordnung 40 steht. Theoretisch könnten gleichzeitig Messungen an allen sechs Messplätzen M1, M2, M3, M4, M5, M6 stattfinden. Eine zentrale Säule 43 hält die Grundfeldmagnet-Segmente 44 an ihrem Platz und kann auch technische Komponenten umfassen, wie z.B. die elektrischen Verbindungen oder sogar die Stromversorgung (siehe z.B. Figur 4) .

An den Messplätzen M1, M2, M3, M4, M5, M6 können sich jeweils Messeinrichtungen 12 (nur jeweils symbolisch dargestellt) bzw. die hierzu am Messplatz M1, M2, M3, M4, M5, M6 jeweils erforderlichen Komponenten befinden, wie eine HF-Sendespule eines HF-Sendesystems, eine HF-Empfangsspule eines HF-Empfangssystem und/oder eine gemeinsame HF-Sende-/Empfangsspule. Ebenso können hierzu Gradienten- und/oder Shimspulen gehören. Alle diese Komponenten können von der gemeinsamen Steuereinrichtung 13 koordiniert angesteuert werden.

Selbstverständlich kann ein Magnetresonanzscanner 2 auch mehr als sechs Messplätze M1, M2, M3, M4, M5, M6 aufweisen, seine Höhe kann geringer sein oder er ist zur Untersuchung kleiner Körperbereiche ausgelegt, z.B. für Kopfuntersuchungen oder Untersuchungen der Extremitäten, der weiblichen Brust, der Prostata, der Leber, Nieren oder von anderen Organen. Auch könnte die sternförmige Grundfeldmagnetanordnung 40 liegend positioniert sein.

Figur 4 zeigt ein Ausführungsbeispiel für ein Magnetresonanztomographie-System 1, mit einer supraleitenden Grundfeldmagnetanordnung 40. Dies könnte eine supraleitende Version der in Figur 2 gezeigten Ausführungsform sein. Zur besseren Darstellung des inneren Aufbaus sind die vorderen beiden Grundfeldmagnet-Segmente 44 nicht dargestellt. Zu sehen ist, dass die Grundfeldmagnetanordnung 40 mit Helium He gefüllt ist, welches teilweise flüssig und teilweise gasförmig ist. Das Referenzzeichen für das Helium He zeigt dabei auf den Flüssigkeitsspiegel. Die gesamte Grundmagnetfeldanordnung 40 ist von einer Gehäusewand 30 umgeben, die hier insbesondere eine thermische Isolierung aufweist, damit das Helium He im Gehäuseinnenraum 33 kalt und damit flüssig bleibt.

Solche eine Isolierung kann z.B. Vielschicht-Isolationsfolien oder thermisch leitfähige Abschirmungen gegen umgebende thermische Strahlung umfassen.

Oben in der Mitte der Grundfeldmagnetanordnung 40 ist eine Kühleinheit 22 angebracht, an deren Kühlfinger 22a ständig Helium He kondensiert und nach unten tropft. Durch eine Heliumleitung 22b kann der Heliuminhalt und der Druck in der Grundfeldmagnetanordnung 40 reguliert werden. Unten ist eine Magnetzuleitungsverteiler 20 (engl.: superconducting joints box) an einer Schalteinheit 23 zu sehen, die über Stromzuführungen 24 die Grundfeldmagnetanordnung 40 mit Strom versorgen kann. Die Schalteinheit 23 kann hier als persistenter Schalter verwendet werden, um einen stetig kreisenden Strom und damit ein permanentes Grundmagnetfeld B0 in der supraleitenden Grundfeldmagnetanordnung 40 zu erzeugen.

Es sind aber auch andere Kühlungsalternativen möglich, z.B. mittels Durchleitung von flüssigem Helium durch Hohlleiter der Magnete oder durch zusätzliche Kühlleitungen in gutem thermischen Kontakt mit den Magnetspulen. Es können in dem Aufbau der der Grundfeldmagnetanordnung 40 noch weitere Komponenten enthalten sein, die der Übersicht halber hier nicht eingezeichnet sind wie z.B. ein Quench-Detektor bzw. -Schutz, ein sogenannter "coil carrier" (Magnetformer) oder strukturelle Verstärkungen.

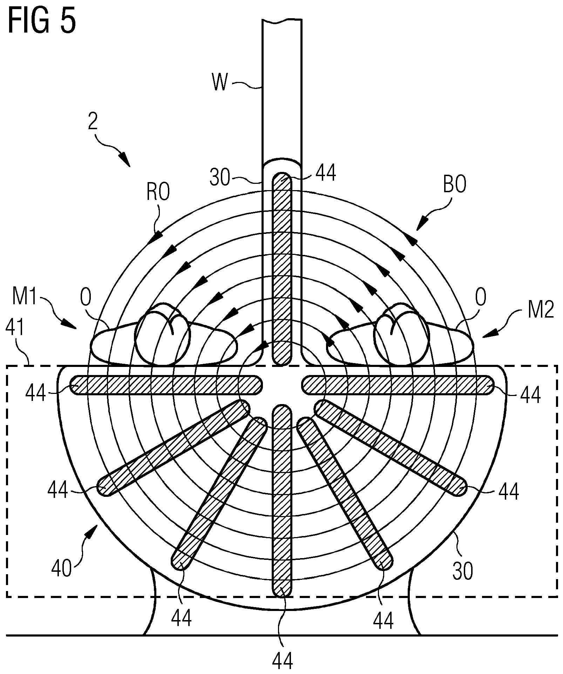

Figur 5 zeigt ein weiteres Ausführungsbeispiel für einen Magnetresonanzscanner 2 mit einer erfindungsgemäßen Grundfeldmagnetanordnung 40. Hier ist nur die untere Hälfte der Grundfeldmagnetanordnung 40 als eine Gruppe 41 von Grundfeldmagnet-Segmenten 44 sternförmig gestaltet und ein weiteres Grundfeldmagnet-Segment 44 ragt nach oben heraus und dient sowohl zur Führung des Grundmagnetfeldes B0 als auch als Teil einer Wandung W zwischen zwei Messplätzen M1, M2, auf denen sich hier zwei Patienten O befinden. In der Darstellung ist zu sehen dass der untere Teil der Wandung W zwischen den beiden Patienten O durch die Gehäusewandung 30 des Magnetresonanzscanners 2 gebildet wird, in die das Grundfeldmagnet-Segment 44 zwischen den Messplätzen M1, M2 integriert ist. Die Wandung W kann nicht nur als ein Sichtschutz dienen, sondern auch als eine akustische Abschirmung bzw. eine HF-Abschirmung.

Das Grundmagnetfeld B0 dieses Magnetresonanzscanners 2 wird nach außen hin schwächer, was zur Ortscodierung genutzt werden kann, und ist in Längsrichtung (orthogonal zur Bildebene) homogen. Es ist im Grunde in den beiden Messplätzen M1, M2 von seiner Form her gleich, mit dem einzigen Unterschied, dass der Verlauf (in einer Richtung durch die Oberfläche, auf der der Patient O gelagert wird) umgekehrt ist. Auch hier können wie bei Figur 2 die Dimensionen des Magnetresonanzscanners 2 durchaus anders gewählt werden.

Die Grundmagnet-Hauptfeldrichtung R0 ist auch hier kreisförmig. Eine Besonderheit bei dieser Ausführungsform ist, dass die Patienten O nicht in einem engen Raum liegen, sondern frei zur Decke schauen können. Die durch die Krümmung in der Regel hervorgerufene Inhomogenität im Grundmagnetfeld B0 kann wie erwähnt zur Ortskodierung einer Raumrichtung verwendet werden, so dass zur Gesamtortskodierung lediglich Gradienten in die andere Raumrichtung appliziert werden müssen.

Diese Anordnung lässt wegen ihrer offenen Bauform und dem toroidalen Magnetfeld einen einfachen und wenig eingeschränkten Zugang zum Patienten zu. Durch den besonderen Aufbau werden Magnetkräfte wie bei der Figur 2 zum großen Teil kompensiert bzw. in Bereiche abgeleitet, die sich gut strukturell verstärken lassen.

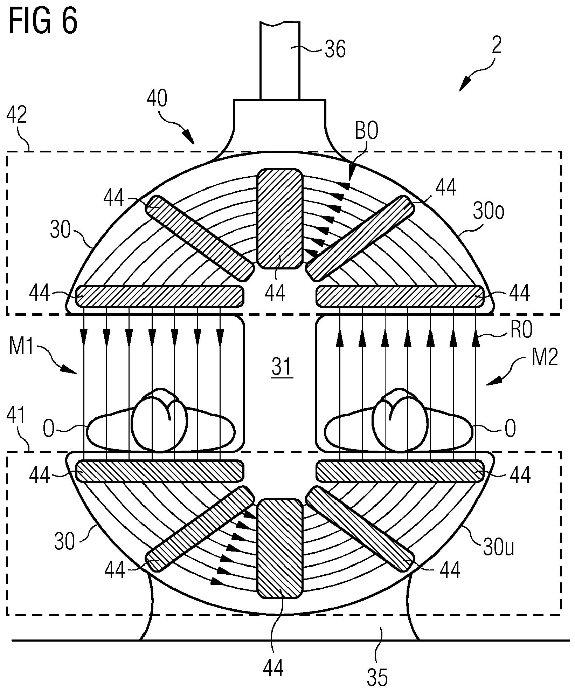

Figur 6 zeigt ein weiteres Ausführungsbeispiel für einen Magnetresonanzscanner 2 mit einer erfindungsgemäßen Grundfeldmagnetanordnung 40. Diese ist ähnlich zu der in Figur 5 aufgebaut, mit dem Unterschied, dass sich nun über und unter den beiden Messplätzen M1, M2 eine Gruppe 41, 42 von Grundfeldmagnet-Segmenten 44 befindet. Wie an dem eingezeichneten Verlauf der Magnetfeldlinien zu sehen ist, ist der Verlauf des bestimmungsgemäßen Grundmagnetfelds B0 hier sehr homogen im Bereich der Messplätze M1, M2.

Das Gehäuse 30 umfasst hier einen oberen und einen unteren halbzylinderförmigen Gehäuseabschnitt 30u, 30o, jeweils mit einem Querschnitt in der Form eines 180° Kreissegmentes, in denen jeweils eine Gruppe 41, 42 von Grundfeldmagnet-Segmenten 44 untergebracht ist. Die Gruppen 41, 42 von Grundfeldmagnet-Segmenten 44 werden mittels eines Mittelstegs bzw. Abgrenzungselements 31, welches Teil des Gehäuses 30 ist, voneinander beabstandet gehalten, wobei die Halbzylinder der Gehäuseabschnitte 30u, 30o jeweils mit ihrer flachen Seite aufeinander zuweisen, wodurch zwei Messeplätze M1, M2 zwischen den Gehäuseabschnitten 30u, 30o bereitgestellt werden. Der untere halbzylinderförmige Gehäuseabschnitt 30u steht auf einem Basisteil 35 und der obere halbzylinderförmige Gehäuseabschnitt 30o kann an einer Deckenhalterung 36 zusätzlich gehalten werden. Das Abgrenzungselement 31 dient dabei gleichzeitig als Trennwand bzw. Wandung W zwischen den beiden Messplätzen M1, M2.

Der hier gezeigte Aufbau hat verglichen mit dem Aufbau der Figur 5 das Ziel, ein homogenes Magnetfeld an den beiden Messplätzen M1, M2 zu erzeugen. Die räumliche Offenheit eines solchen Magnetresonanzscanners 2 ist vergleichbar mit einem C-Magnetsystem, jedoch ist im Unterschied zu einem solchen kein massives Eisenjoch zur Abschirmung bzw. Umlenkung der Magnetfeldlinien vonnöten. Stattdessen wird die Gruppe 41, 42 von Grundfeldmagnet-Segmenten 44 zur Abschirmung und Lenkung des Grundmagnetfeldes B0 verwendet, was das Gewicht gravierend reduziert.

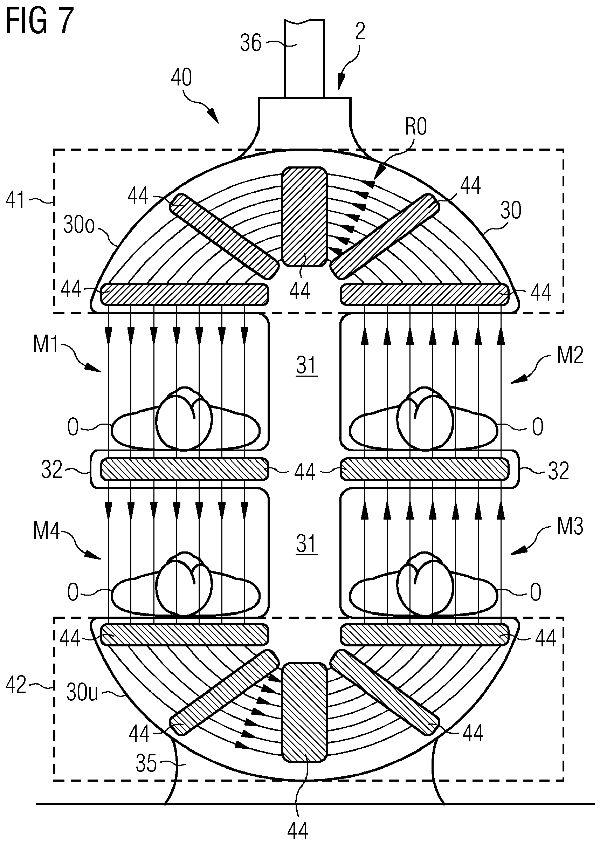

Figur 7 zeigt ein weiteres Ausführungsbeispiel für ein Magnetresonanztomographie-System 1 mit einer erfindungsgemäßen Grundfeldmagnetanordnung 40. Dieses ähnelt sehr dem Aufbau der Figur 6 mit dem Unterschied, dass hier an Stelle von zwei nun vier Messplätze M1, M2, M3, M4 vorhanden sind. Jeweils zwei der Messplätze M1, M2, M3, M4 sind übereinander angeordnet, wobei der jeweils obere vom unteren durch ein Bodenelement 32 des Gehäuses 30 abgetrennt ist. Dieses Bodenelement 32 dient gleichzeitig als Aufnahme eines Grundfeldmagnet-Segmentes 44, welches das Magnetfeld in dem Magnetresonanzscanner 2 homogen durch die Messplätze M1, M2, M3, M4 führt. Die Bodenelemente 32 können beispielsweise an dem als Trennwand bzw. Wandung W zwischen den jeweils benachbarten Messplätzen M1, M2, M3, M4 dienenden Mittelsteg bzw. Abgrenzungselement 31 des Gehäuses 30 verankert sein bzw. sich seitlich aus dieser heraus erstrecken.

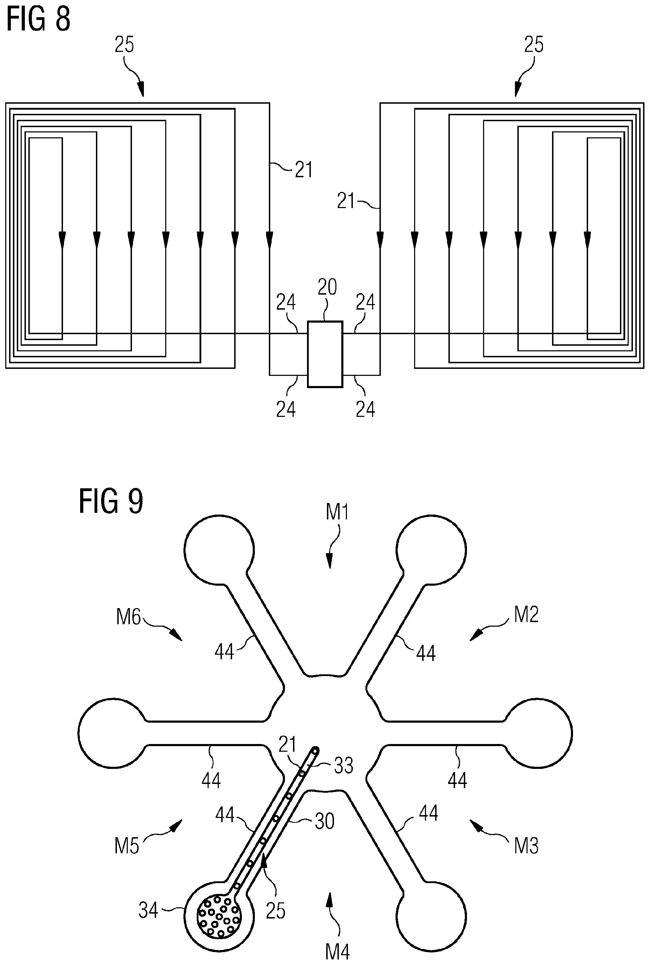

Figur 8 zeigt eine schematische Darstellung von Spulenwicklungen 25 aus Stromleitern 21 zur Kompensation von Inhomogenitäten in einem ringförmigen Magnetfeld, wie sie im Rahmen der Erfindung eingesetzt werden können. Man kann sich vorstellen, dass diese Wicklungen in gegenüberliegenden Grundfeldmagnet-Segmenten 44 in Figur 3 vorliegen. Wie man deutlich erkennen kann, sind die Spulenwicklungen 25 so gestaltet, dass der Durchmesser einer Wicklung in einer Raumrichtung (nämlich hier der Horizontalen), verglichen mit ihrer benachbarten Wicklung, abnimmt und ihr Mittelpunkt dichter an einem Außenrand des ringförmigen Grundmagnetfelds B0 liegt als der Mittelpunkt der größeren Wicklung. Dadurch wird ein bestimmungsgemäßes Magnetfeld (eines jeweiligen Grundfeldmagnet-Segments 44) erzeugt, das jeweils zu den Außenseiten der Grundfeldmagnet-Segmente 44 hin stärker wird. Dadurch kann - bei geeignetem Aufbau - die im Zusammenhang mit den Figuren 2, 3 und 5 beschriebene, in radialer Richtung verlaufende Inhomogenität des Grundmagnetfelds B0 zumindest teilweise, vorzugsweise vollständig, ausgeglichen werden.

Figur 9 zeigt von oben eine schematische Darstellung einer in Figur 2 dargestellten Ausführungsform mit einer Spulenwicklung 25 nach Figur 8. Hier ist nur ein Grundfeldmagnet-Segment 44 mit einer Detailzeichnung versehen. Es sollte davon ausgegangen werden, dass auch alle anderen Grundfeldmagnet-Segmente 44 solchermaßen aufgebaut sind.

Die Dichte der Stromleiter 21 kann auch mit zunehmenden radialen Abstand zunehmen.

Die Spulenwicklung 25 ist in einem Gehäuseinnenraum 33 des Gehäuses 30 untergebracht. Da die Anzahl der Stromleiter 21 (der Übersichtlichkeit halber ist nur einer bezeichnet) am Rand sehr hoch ist, und ansonsten die Dichte regelmäßig über die Breite eines Grundfeldmagnet-Segments 44 ist, weist diese Grundfeldmagnetanordnung 40 an den äußeren Endabschnitten 34 der Seitenwände der Grundfeldmagnet-Segmente 44 Verdickungen auf, in denen die Stromleiter gebündelt geführt werden.

Es wird abschließend noch einmal darauf hingewiesen, dass es sich bei den vorhergehend detailliert beschriebenen Ausführungsformen sowie bei dem dargestellten Magnetresonanztomographie-System 1 bzw. der Grundfeldmagnetanordnung 40 lediglich um Ausführungsbeispiele handelt, welche vom Fachmann in verschiedenster Weise modifiziert werden können, ohne den Bereich der Erfindung zu verlassen. So ist klar, dass beispielsweise sämtliche dargestellte Magnetresonanzscanner bzw. MR-Systeme mit entsprechenden Steuereinrichtungen und anderen zusätzlichen Komponenten ausgestattet sein können, wie sie beispielsweise in den Figuren 1 und/oder 2 dargestellt werden, auch wenn diese in den Figuren nicht jeweils explizit gezeigt sind. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Anordnung" nicht aus, dass die betreffenden Komponenten aus mehreren zusammenwirkenden Teil-Komponenten bestehen, die gegebenenfalls auch räumlich verteilt sein können.

## Patentansprüche

1. Grundfeldmagnetanordnung (40) für ein Magnetresonanztomographie-System (1),
welche Grundfeldmagnetanordnung (40) mehrere räumlich voneinander getrennte Grundfeldmagnet-Segmente (44) aufweist, um jeweils ein bestimmungsgemäßes Magnetfeld mit einer definierten Segment-Hauptfeldrichtung (R1) zu erzeugen,
wobei zumindest zwei der Grundfeldmagnet-Segmente (44) so zueinander angeordnet sind, dass die Segment-Hauptfeldrichtungen (R1) ihrer bestimmungsgemäßen Magnetfelder unter einem Ablenkwinkel zueinander verlaufen, so dass die bestimmungsgemäßen Magnetfelder der Grundfeldmagnet-Segmente (44) in einem bestimmungsgemäßen Grundmagnetfeld (B0) resultieren, wobei das Grundmagnetfeld (B0) eine Grundmagnet-Hauptfeldrichtung (R0) mit einem ringförmigen Verlauf aufweist.

2. Grundfeldmagnetanordnung nach Anspruch 1, umfassend mindestens drei Grundfeldmagnet-Segmente (44), welche so angeordnet sind, dass die Grundmagnet-Hauptfeldrichtung (R0) die Form eines ebenen Rings aufweist, bevorzugt eines Toroids oder die Form eines Toroids mit eingefügten geraden Passagen.

3. Grundfeldmagnetanordnung nach einem der vorangehenden Ansprüche, wobei der Ablenkwinkel der Segment-Hauptfeldrichtungen der Magnetfelder zumindest zweier benachbarter Grundfeldmagnet-Segmente (44) mindestens 5°, bevorzugt mindestens 30°, besonders bevorzugt mindestens 45°, beträgt.

4. Grundfeldmagnetanordnung nach einem der vorangehenden Ansprüche, umfassend zumindest eine Gruppe (41, 42) von Grundfeldmagnet-Segmenten (44), die sternförmig um zumindest eine Mittelachse (A) herum angeordnet sind, bevorzugt drehsymmetrisch,
wobei besonders bevorzugt bei N Grundfeldmagnet-Segmenten (44) eine Drehsymmetrie von 360°/N vorliegt.

5. Grundfeldmagnetanordnung nach einem der vorangehenden Ansprüche, umfassend ein Grundfeldmagnet-Segment (44) oder eine Gruppe (41, 42) von Grundfeldmagnet-Segmenten (44), welche aufgebaut ist, um die Grundmagnet-Hauptfeldrichtung (R0) des bestimmungsgemäßen Grundmagnetfelds (B0) um einen Gesamt-Ablenkwinkel von mindestens 60°, bevorzugt von mindestens 90°, weiter bevorzugt von mindestens 180° abzulenken.

6. Grundfeldmagnetanordnung nach einem der vorangehenden Ansprüche, wobei ein Grundfeldmagnet-Segment (44) eine Spulenwicklung (25) aufweist, die ein bestimmungsgemäßes Magnetfeld erzeugt, das in einer Richtung quer zur Segment-Hauptfeldrichtung (R1) des Grundfeldmagnet-Segments (44) hin stärker wird,
wobei die Spulenwicklung (25) bevorzugt so gestaltet ist, dass der Durchmesser einer Wicklung in zumindest einer Raumrichtung, verglichen mit ihrer benachbarten Wicklung, abnimmt und ihr Mittelpunkt dichter an einer äußeren Seitenwand (34) des Grundfeldmagnet-Segments (44) liegt.

7. Magnetresonanztomographie-System (1) umfassend eine Grundfeldmagnetanordnung (40) nach einem der vorangehenden Ansprüche.

8. Magnetresonanztomographie-System (1) nach Anspruch 7, umfassend eine Mehrzahl von Messplätzen (M1, M2, M3, M4, M5, M6) innerhalb des bestimmungsgemäßen Grundmagnetfelds,
wobei die Messplätze (M1, M2, M3, M4, M5, M6) vorzugsweise zwischen jeweils zwei Grundfeldmagnet-Segmenten (44) und/oder in einem Patiententunnel (3) innerhalb eines Grundfeldmagnet-Segments (44) angeordnet sind,
wobei ein Grundfeldmagnet-Segment (44) bevorzugt als Wandung (W) ausgebildet ist und/oder in einer Wandung (W) zwischen zwei Messplätzen (M1, M2, M3, M4, M5, M6) angeordnet ist.

9. Magnetresonanztomographie-System (1) nach Anspruch 8, wobei zumindest zwei Messplätze (M1, M2, M3, M4, M5, M6) in Bereichen mit einer gegenläufigen Grundmagnet-Hauptfeldrichtung (R0) angeordnet sind, wobei die Messplätze (M1, M2, M3, M4, M5, M6) bevorzugt nebeneinander angeordnet sind und besonders bevorzugt auf einer Ebene liegen.

10. Magnetresonanztomographie-System (1) nach einem der Ansprüche 7 bis 9, wobei die Grundfeldmagnetanordnung (40) ein Grundfeldmagnet-Segment (44) oder eine Gruppe (41, 41) von Grundfeldmagnet-Segmenten (44) aufweist, welche aufgebaut ist um die Grundmagnet-Hauptfeldrichtung (R0) des bestimmungsgemäßen Grundmagnetfelds (B0) um einen Gesamt-Ablenkwinkel von mindestens 60°, bevorzugt von mindestens 90°, weiter bevorzugt von mindestens 180° abzulenken,
wobei dieses Grundfeldmagnet-Segment (44) oder die Gruppe (41, 41) von Grundfeldmagnet-Segmenten (44) unter oder über zumindest einem Messplatz (M1, M2, M3, M4, M5, M6) oder an zumindest einer Seite eines Messplatzes (M1, M2, M3, M4, M5, M6) angeordnet ist,
wobei vorzugsweise dieses Grundfeldmagnet-Segment (44) oder die Gruppe (41, 42) von Grundfeldmagnet-Segmenten (44) an zwei unterschiedliche Messplätze (M1, M2, M3, M4, M5, M6) angrenzt.

11. Magnetresonanztomographie-System (1) nach einem der Ansprüche 7 bis 10, umfassend eine Mehrzahl von, vorzugsweise voneinander unabhängigen, Messeinrichtungen (12), wobei jede der Messeinrichtungen (12) zur Durchführung einer Messung im Rahmen einer Magnetresonanztomographiebildgebung an einem der Messplätze (M1, M2, M3, M4, M5, M6) ausgelegt ist.

12. Verfahren zur Messung von Rohdaten für eine Magnetresonanztomographieaufnahme, umfassend die Schritte:
- Positionieren eines Objekts in einem Messplatz eines Magnetresonanztomographie-Systems (1) nach einem der Ansprüche 7 bis 11,
- Erzeugen eines Grundmagnetfelds (B0) mit der Grundfeldmagnetanordnung (40) des Magnetresonanztomographie-Systems (1),
- Messung der Rohdaten.

13. Verfahren nach Anspruch 12, wobei eine Inhomogenität des das Grundmagnetfelds (B0), insbesondere durch Ausnutzung des ringförmigen Verlaufs der Grundmagnet-Hauptfeldrichtung (R0), zur Ortskodierung der Rohdaten genutzt wird.

14. Verfahren nach Anspruch 12 oder 13, wobei an zumindest zwei Messplätzen (M1, M2, M3, M4, M5, M6) gleichzeitig Magnetresonanztomographieaufnahmen durchgeführt werden.

15. Verwendung eines toroidalen Grundmagnetfelds (B0) für eine Messung im Rahmen der Magnetresonanztomographie und/oder in einem Resonanztomographie-System (1), vorzugsweise an mehr als einem Messplatz (M1, M2, M3, M4, M5, M6) innerhalb eines gemeinsamen toroidalen Grundmagnetfelds (B0).
